# EUROPEAN PATENT APPLICATION

(11) **EP 3 406 592 A1**
(43) Date of publication of application: **28.11.2018**
(21) Application number: 17172612.8
(22) Date of filing: 24.05.2017
(51) Int. Cl.: C07C 317/00, C08G 65/40, C08G 75/23, C08K 7/00

(54) **DICHLORODIPHENYL SULFONE PASTILLES**

(71) Applicant: Solvay Specialty Polymers USA, LLC., Alpharetta, GA 30005-3914 (US)
(72) Inventor: DOSI, Mahendra K., Alpharetta, GA Georgia 30022 (US); HUSEIN, Ziad, Evans, GA Georgia 30809 (US); MYSONA, Ronald, Evans, GA Georgia 30809 (US)
(74) Representative: Fiorucci, Hélène

(57) **Abstract**

Described herein are pastilles comprising a dicholorodiphenyl sulfone ("DCDPS"), corresponding formation methods and methods of synthesizing poly(aryl ether sulfone)s incorporating the DCDPS pastilles. It was surprisingly discovered that the use of pastilles comprising DCDPS as monomer source significantly increased the charge rate of the monomer into polymerization reactors during industrial scale poly(aryl ether sulfone) ("PAES") synthesis. The DCDPS pastilles can be formed by specifically adapted deposition approaches to achieve selected pastilles sizes with a narrow size distribution and, furthermore, can be desirably used in large scale PAES polymer synthesis to significantly improve polymer throughput.

## Description

### FIELD OF THE INVENTION

The invention relates to dichlorodiphenyl sulfone pastilles. The invention further relates to methods of making dichlorodiphenyl sulfone pastilles and methods of poly(aryl ether sulfone) polymer synthesis incorporating dichlorodiphenyl sulfone pastilles.

### BACKGROUND OF THE INVENTION

Industrial scale polymer synthesis requires high throughput to meet customer demands. In any such synthesis, the rate at which monomer components are delivered to the polymerization reactor can place significant limits on the production rate of the polymer. For example, based upon the monomer composition and morphology used, flow rates of the monomer to the polymerization reactor may be limited due to partial or total clogging of pipes conveying the monomer to the polymerization reactor. Poly(aryl ether sulfone) ("PAES") polymer synthesis involves dichlorodiphenyl sulfone ("DCDPS") as a starting monomer for the polymerization reaction. Accordingly, improvements in the deliver rate of the DCDPS monomer to the polymerization reactor can provide significant improvements in the overall production rate of the PAES polymer.

### BRIEF DESCRIPTION OF THE DRAWINGS

Fig. 1a is a schematic depiction of a side view of a DCDPS pastille.
Fig. 1b is a schematic depiction of the top view of a DCDPS pastille.
Fig. 2 is a schematic depiction of an apparatus for forming DCDPS pastilles
Fig. 3 is a schematic depiction of a batch polymerization system for the production of PAES polymers.
Fig. 4 is a schematic depiction of an apparatus for measuring the angle of repose using the tilting box method.

### DETAILED DESCRIPTION OF THE INVENTION

Described herein are pastilles comprising a dichlorodiphenyl sulfone ("DCDPS"), corresponding formation methods and methods of synthesizing poly(aryl ether sulfone)s incorporating the DCDPS pastilles. It was surprisingly discovered that the use of pastilles comprising DCDPS as monomer source significantly increased the charge rate of the monomer into polymerization reactors during industrial scale poly(aryl ether sulfone) ("PAES") synthesis. The DCDPS pastilles can be formed by specifically adapted deposition approaches to achieve selected pastilles sizes with a narrow size distribution and, furthermore, can be desirably used in large scale PAES polymer synthesis to significantly improve polymer throughput.

Powder formation during DCDPS handling can introduce significant difficulties into PAES processing and synthesis. DCDPS particles in a powder are generally present as part of an agglomerated mass, due to short range van der Waals interactions and moisture uptake form the ambient atmosphere. As used herein, a DCDPS powder refers to a collection of DCDPS particles having an average primary (un-agglomerated) particle diameter of from about 5 micrometers ("µm") to 1 mm, preferably to 800 µm, more preferably to 500 µm. Moreover, the extent of agglomeration can increase significantly during relative short storage times (e.g. three weeks). While PAES synthesis generally involves a DCDPS monomer source in the form of granules (e.g. generally spherical having a diameter of from about 2 millimeters ("mm") to about 5 mm), significant quantities of DCDPS powders are also present, due to attrition (breakage or cracking of the DCDPS granules) during shipping, storage, and handling. Furthermore, in large scale PAES synthesis, the DCDPS granules are conveyed from a storage bin to the polymerization reaction vessel, during which further DCDPS powder generation occurs as described above. The DCDPS powders (e.g. due to attritioning) tend to agglomerate and reduce the flow of the DCDPS granules to the polymerization reactor because it promotes clogging of transport pathways (*e.g*. pipes) connecting the DCDPS storage vessel with the polymerization reactor. Correspondingly, PAES synthesis is generally performed at a relatively reduced rate to accommodate the reduced DCDPS flow (and to attempt to lessen the amount of clogging). Additionally, any solid flow blockage can upset the stoichiometric ratio control of the monomer feeds as well. The reduced production concomitantly increases the storage time of the DCDPS monomer source, exacerbating the problem of powder agglomeration.

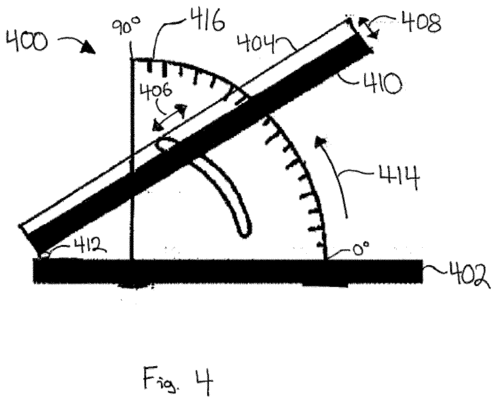

It was surprisingly found that the use of DCDPS pastilles in the DCDPS processing allowed for increased DCDPS monomer flow and correspondingly increased production throughput of PAES polymers during industrial scale synthesis. The DCDPS pastilles described herein have increased structural integrity relative to DCDPS granules, resulting in significantly less generation of powders (due to attrition) and a significantly increased monomer flow rate from the DCDPS storage vessel to the PAES polymerization reactor. The flowability of the DCDPS pastilles can be measured by measuring the angle of repose. The DCDPS pastilles described herein have an angle of repose of no more than 35 degrees or no more than 30 degrees. In additional or alternative embodiments, the DCDPS pastilles have an angle of repose of at least about 10 degrees, at least about 15 degrees, or at least about 20 degrees. The angle of repose can be measured using the tilting box method. Fig. 4 is a schematic depiction of an apparatus for measuring the angle of repose using the tilting box method. Tilting box apparatus 400 includes level surface 402 and tilt box 404. Tilt box 404 is made of clear acrylic and has a length of 155 mm a width of 105 mm and a height of 20 mm. The dimensions of the length and height of tilt box 404 are indicated by double-headed arrows 406 and 408, respectively. The dimension of the width is perpendicular to the dimensions of the length and the width. When tilt box 404 and level surface 402 are in contact (parallel to each other, 0°), 25 g of sample 410 is evenly distributed in tilt box 404 to form a substantially flat layer. Tilt box 404 is rotated about axis 412 (perpendicular to the dimension of length and width) along direction 414 at a rate of 3 °/second. When sample 410 began to slide by visual detection, the rotation of tilt box 404 was stopped and the angle between the bottom of tilt box 404 and level surface 402 was measured using angle measurement device 416. Measuring device 412 can be an inclinometer, protractor or any other device known in the art as suitable for measuring angles. The measurement procedure is done 3 times for each sample 410, and the results are averaged.

Furthermore, it was discovered that DCDPS pastilles having selected sizes and relatively narrow size distributions could be formed using specifically adapted processing approaches. Traditionally, pastille formation involves processing of higher viscosity material, for which control of pastille size is easily obtainable using a deposition approach because of the reduced flow rate material. However, low viscosity organic material, such as DCDPS, presents significant processing challenges during deposition because of the increased flow of the molten material. The increased flow can translate into undesirable loss of control on the physical dimensions of the formed pastilles. As described in detail below, it was found that by implementing a specifically adapted deposition to control the solidification rate of DCDPS during pastille formation, tight control of pastille size and size distribution can be achieved.

### The Dichlorodiphenyl sulfone Pastilles

The pastilles of interest herein comprises at least 50 wt% of a DCDPS, relative to the total weight of the pastille. In some embodiments, the pastilles contain at least 60 wt.%, at least 65 wt.%, at least 70 wt.%, at least 75 wt.%, at least 80 wt.%, at least 85 wt.%, at least 90 wt.%, at least 99 wt.%, at least 99.9 wt.% or at least 99.99 wt.% of the DCDPS.

DCDPS is represented by the following formula: 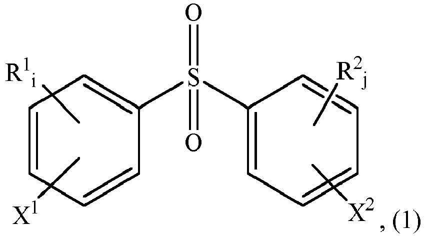 where R¹ and R², at each instance, are independently selected from the group consisting of a halogen, an alkyl, an alkenyl, an alkynyl, an aryl, an ether, a thioether, a carboxylic acid, an ester, an amide, an imide, an alkali or an alkaline earth metal sulfonate, an alkyl sulfonate, an alkali or alkaline earth metal phosphonate, an alkyl phosphonate, an amine and a quaternary ammonium; i and j are independently selected integers from 0 to 4, and X¹ and X² are independently selected halogens. As used herein, a halogen refers to any one of F, Cl, Br, I and At. In some embodiments, the DCDPS is represented by the following formula: 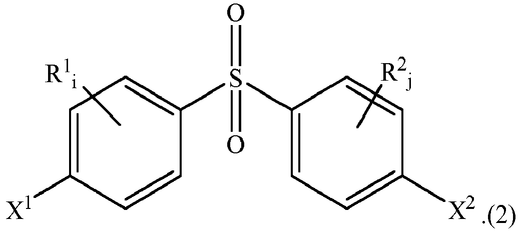 In some such embodiments, i and j are zero. In additional or alternative embodiments, X¹ and X² are independently selected from Cl and F, preferably X¹ and X² and Cl.

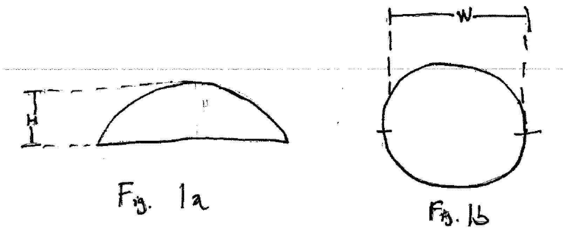

The pastilles have a hemispherical or compressed hemispherical morphology. Referring to Fig. 1, pastille 100 can be characterized by a height (*e.g.* major axis perpendicular to the plane of the base), H, and a width (*e.g.* major axis in the plane of the base), W. For hemispherical pastilles, the height and width can both be defined by the radius of the hemisphere. Compressed hemispheres have a curvature of surface 102 that is, on average, less than the curvature of a hemisphere having a radius equal to the height of the compressed hemisphere. A collection of pastilles can have an average height of at least about 0.1 mm, at least about 0.5 mm, at least about 0.6 mm, at least about 0.7 mm, at least about 0.8 mm, or at least about 0.9 mm. Additionally or alternatively, the pastilles in the collection of pastilles have an average height of from about 1 mm to about 4 mm, to about 3 mm or to about 2 mm. Additionally or alternatively, the pastilles can have an average width of from about 1 mm to about 5 mm or from 2 mm to about 4 mm. In some embodiments, the ratio of the width to height ("aspect ratio") for a collection of pastilles can be from about 1.8 to about 2.2.

A collection of pastilles can have a relative narrow size distribution, in conjunction with the average sizes (height, width and aspect ratio) described above. In some embodiments, at least about 90 percent, at least about 95 percent, at least about 97% or at least about 99% of the individual pastilles in a collection of pastilles have a height greater than about 80 percent of the average width and less than about 120 percent of the average width. In some embodiments, at least about 90 percent, at least about 95 percent, at least about 97% or at least about 99% of the individual pastilles in a collection of pastilles have a height greater than about 80 percent of the average height and less than about 120 percent of the average height. In some embodiments, at least about 90 percent, at least about 95 percent, at least about 97% or at least about 99% of the individual pastilles in a collection of pastilles have an aspect ratio greater than about 80 percent of the average aspect ratio and less than about 120 percent of the average aspect ratio.

In some embodiments, the collection of pastilles can have a weight of at least about 0.5 kg, at least about 1 kg, at least about 5 kg, at least about 10 kg, at least about 20 kg, at least about 40 kg, at least about 100 kg or at least about 200 kg. In some embodiments, the collection of pastilles can have a weight of from about 0.45 kg to about 45 kg, from about 4.5 kg to about 45 kg or from about 4.5 kg to about 22.6 kg. In some embodiments, the collection of pastilles can have a weight of from about 135 kg to about 275 kg.

As noted above, the DCDPS pastilles have increased structural integrity and, correspondingly, powder generation from breakage during handling is reduced, relative to DCDPS granules. In some embodiments, a collection of DCDPS pastilles can have a DCDPS powder content of no more than about no more than about 5 wt.%, no more than about 4 wt.%, no more than about 3 wt.%, no more than about 2 wt.% or no more than about 1 wt.%, relative to the total weight of the DCDPS pastilles and DCDPS powder.

### Formation of DCDPS Pastilles

The pastilles described herein can be formed by a deposition process whereby liquid (*e.g*. molten) DCDPS is discretely deposited onto a surface to form DCDPS pastilles. In general, the temperature difference between the liquid DCDPS prior to deposition and the temperature of the deposition surface at least partially determines the rate of solidification (during cooling) of the pastille after it is deposited on the surface. If the deposited DCDPS solidifies too quickly, the resulting pastille can have undesirable amounts of internal stress. Larger internal stress can translate into breakage during transportation and handling, resulting in powder generation and difficulties during PAES synthesis, as described in detail above. If the deposited DCDPS solidifies too slowly, tight control of the pastille dimensions (and corresponding size distribution in a collection of pastilles) is sacrificed due to the increased spread time of the DCDPS on the surface after deposition.

Applicant found that by appropriate selection of the liquid DCDPS temperature and the deposition surface temperature, DCDPS pastilles having a narrow size distribution and desirable structural integrity can be formed. For the deposition processes of interest herein, the temperature of the liquid DCDPS is from about Tm + 5° C to about Tm + 35° C, where Tm is the melting point of DCDPS. Tm of DCDPS is about 148° C. In some embodiments, the temperature of the liquid DCDPS is from about 155 ° C to about 180° C. The temperature of the deposition surface can be is from about Tm - 80 ° C to Tm - 110°C or to Tm - 100 °C.

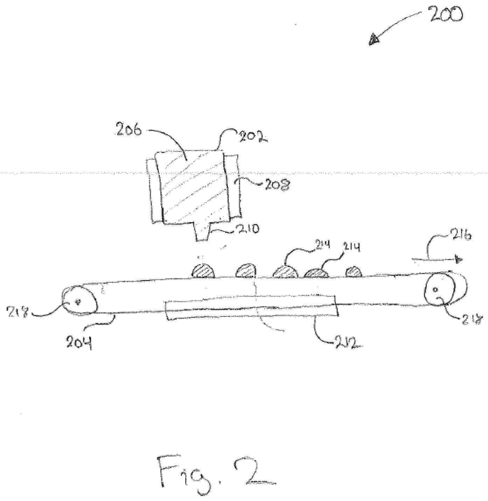

Fig. 2 shows an embodiment of DCDPS formation apparatus. Formation apparatus 200 includes monomer reservoir 202 and conveyor belt 204. Monomer reservoir 202 contains liquid DCDPS 206, held at a selected temperature by heating element 208. A portion of liquid DCDPS 206 is dispensed from nozzle 210 onto conveyor belt 204. Conveyor belt is maintained at a selected temperature by cooling element 212. In some embodiments, cooling element 212 can be a water bath or refrigeration device that blows cold air onto conveyor belt 204. In other embodiments, a stream of water can be sprayed onto the belt to maintain the belt at a constant temperature. DCDPS pastilles 214 are formed by solidification as they travel with conveyor belt 204 in the direction 216 (for clarity, not all pastilles are labelled), where conveyor belt 204 is propelled by rollers 218.

### PAES Polymer and Synthesis

The synthesis of PAES polymers involves the condensation reaction of a DCDPS monomer with a diol. As used herein, a PAES polymer refers to any polymer having at least 50 mole % ("mol%") recurring units (R_{PAES}) according to the following formula: 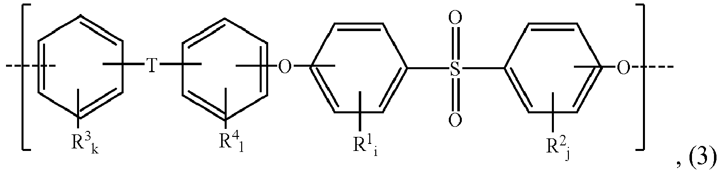 where R³ and R⁴, at each instance, are independently selected from a halogen, an alkyl, an alkenyl, an alkynyl, an aryl, an ether, a thioether, a carboxylic acid, an ester, an amide, an imide, an alkali or alkaline earth metal sulfonate, an alkyl sulfonate, an alkali or alkaline earth metal phosphonate, an alkyl phosphonate, an amine, and a quaternary ammonium; k and l are integers ranging from 0 to 4; and T is selected from the group consisting of a bond, a sulfone group [S(=O)2], and a group -C(R⁵)(R⁶)-, where R⁵ and R⁶ are independently selected from a hydrogen, a halogen, an alkyl, an alkenyl, an alkynyl, an ether, a thioether, a carboxylic acid, an ester, an amide, an imide, an alkali or alkaline earth metal sulfonate, an alkyl sulfonate, an alkali or alkaline earth metal phosphonate, an alkyl phosphonate, an amine, and a quaternary ammonium. Preferably, R⁵ and R⁶ are preferably methyl groups. In formula (3), R¹, R², i and j are the same as those selected in Formulas (1) and (2). Namely, the Formula (3) reflects the fact that the PAES polymer is formed from the polycondensation of the DCDPS monomer and a diol, as explained in detail below. As used herein, the dashed bonds represent a bond to an adjacent recurring unit. In some embodiments, the PAES polymer has at least 60 mol%, at least 70 mol%, at least 80 mol%, at least 90 mol%, at least 95 mol% or at least 99 mol% recurring units (R_{PAES}).

In some embodiments, the PAES polymer is represented by the following formula: 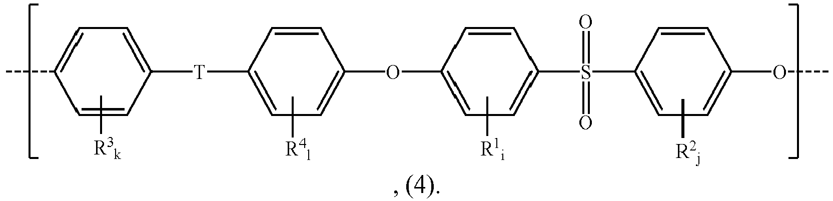 In some embodiments in which recurring unit (R_{PAES}) is represented by Formula (3) or (4), T is a bond (a polyphenylsulfone), -S(=O)₂- (a polyether sulfone) or - C(CH₃)₂- (a polysulfone). In some such embodiments, or in other embodiments according to Formula (3) or (4), k and l are zero; i and j are zero; or i, j, k and l are zero.

The recurring unit (R_{PAES}) of the PAES polymer are formed by the polycondensation reaction of the DCDPS monomer and a diol monomer of the following formula: 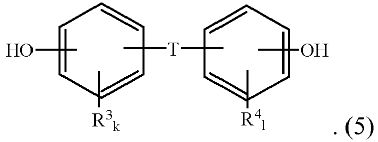 In some embodiments, the diol can be represented by the following formula: 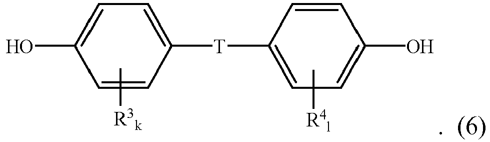 The person of ordinary skill in the art will immediately recognize that the polycondensation of a DCDPS monomer according to Formula (1) and a diol monomer according to Formula (5) results in recurring unit (R_{PAES}) according to Formula (3). The person of ordinary skill in the art will further immediately recognize that the polydondensation of a DCDPS monomer according to Formula (2) and a diol monomer according to Formula (6) result in recurring unit (R_{PAES}) according to Formula (4).

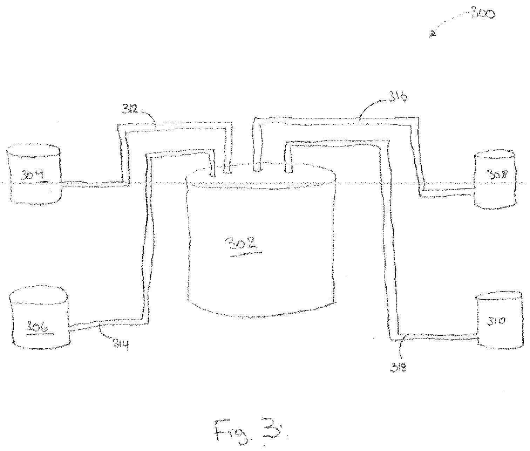

Synthesis of PAES polymers on the industrial scale involves batch processing, in which in which the monomers are stored in storage vessels and transported to a polymerization reactor for each batch polymerization reaction. Fig. 3 is a schematic diagram of a batch polymerization system for PAES synthesis. Referring to Fig. 3, the batch polymerization system 300 includes polymerization reactor 302, DCDPS storage vessel 304, and diol storage vessel 306. Batch polymerization system 300 also includes solvent storage vessel 308 and activator vessel 310. In one synthesis approach, DCDPS monomer, diol monomer, solvent, and activator are delivered from DCDPS storage vessel 304, diol storage vessel 306, solvent storage vessel 308 and activator vessel 310, respectively, to polymerization reactor 302. DCDPS monomer is delivered through pipe 312, and the dial, solvent and activator are delivered through pipes 314, 316 and 318, respectively. The polycondensation reaction is performed by heating the contents of polymerization reactor to the reaction temperature (about 180° C to about 240 ° C). With respect to the form of the DCDPS monomer, clogging (and reduced monomer flow) due to agglomerated DCDPS powder can occur in pipe 312. As noted above, powder in the DCDPS monomer source can be significantly reduced (and correspondingly DCDPS flow improved) by conveying DCDPS pastilles to polymerization reactor 302 through pipe 312.

### EXAMPLES

The following examples demonstrate the flow of DCDPS pastilles.

### Example 1: Formation of DCDPS Pastilles

This example demonstrates the formation of DCDPS pastilles.

To demonstrate performance, dichlorodiphenyl sulfone ("DCDPS") pastilles were formed using an apparatus similar to that depicted in Fig. 2. In particular, liquid DCDPS, stored in a heated reservoir and maintained at a temperature from about 160° C to about 169° C, was metered through a nozzle and deposited onto a conveyor belt, maintained at a temperature from about 26° C to about 31° C or to about 45° C to form the pastilles. The pastilles were collected from the conveyor belt and their dimensions were measured. For each batch, about 20,000 kg of DCDPS pastilles were formed. The average height and width of the pastilles was measured using a Vernier caliper, sampling 30 randomly selected pastilles from the batch.

**Table 1**

| Batch No. | Minimum Width - Maximum Width (mm) | Average Width (mm) | Minimum Height - Maximum Height (mm) | Average Height (mm) |
|---|---|---|---|---|
| 1 | 2.6 - 3.0 | 2.8 | 1.1 - 1.6 | 1.35 |
| 2 | 2.7 - 3.5 | 3.1 | 1.3 - 1.6 | 1.45 |
| 3 | 2.5 - 3.1 | 2.8 | 1.2 - 1.7 | 1.45 |
| 4 | 2.5 - 3.0 | 2.75 | 1.3 - 1.6 | 1.45 |

Referring to Table 1, for the batches processed, excellent control of size (height and width) was obtained, leading to narrow size distributions of the formed DCDPS pastilles. For example, the average widths of the collections of pastilles from batches 1 to 4 were 2.8 mm, 3.1 mm, 2.8 mm and 2.75 mm, respectively. In each case, 100% of the pastilles had a width that was greater than about 80% of the average width and less than about 120% of the average width. Similar results were observed for the height measurements.

### Example 2: Flow Performance of DCDPS Pastilles

This example demonstrates the improved flow performance of DCDPS pastilles relative to DCDPS powders.

To demonstrate flow several samples were formed. Sample 1 was a DCDPS powder. Sample 2 was a collection of DCDPS pastilles, formed as described in Example 1. Samples 3 to 5 consisted of a mixture of DCDPS granules and attrited sulfone granules. The mixture was reflective of the amount of attrition in DCDPS granules. Samples 3 to 5 respectively contained 70 wt.%, 65, wt.% and 55 wt.% DCDPS granules, relative to the total weight of DCDPS in the sample. The DCDPS granules had an average diameter of more than about 4 mm. The balance of each sample consisted of DCDPS powder consisting of DCDPS particles having an average diameter of less than about 0.8.

Flow was determined by measuring the angle of repose of the sample collection of pastilles. The angle of repose was measured using the tilt-box method, as described in detail above. The sample parameters and angle of repose measurements are displayed in Table 2.

**Table 2**

| Sample No. | Pastille Concentration (wt.%) | Granule Concentration (wt.%) | Powder Concentration (wt.%) | Angle of Repose (degrees) |
|---|---|---|---|---|
| 1 | 0 | 0 | 100 | ≥ 90 |
| 2 | 100 | 0 | 0 | 30 |
| 3 | 0 | 70 | 30 | 45 |
| 4 | 0 | 65 | 35 | 40 |
| 5 | 0 | 55 | 45 | 45 |

Referring to Table 2, the flowability of the pure pastilles (sample 2) was significantly larger, relative to pure powder (sample 1) and granule/powder mixtures (samples 3 to 5).

### Example 3: Flow Performance of DCDPS Pastilles in Industrial Scale Processing

The example demonstrates the improved flow performance of DCDPS pastilles in an industrial scale PAES synthesis system.

To demonstrate flow, DCDPS was loaded into a storage bin a delivered, through a pipe, to a polymerization reactor. The DCDPS was transported through the pipe using air. The configuration was similar to the PAES synthesis apparatus schematically depicted in Fig. 3. Three batches were run. Batch 5 and batch 6 consisted of a 1:1 weight ratio of DCDPS granules to DCDPS pastilles. Batch 7 consisted of DCDPS pastilles alone. For each batch, the DCDPS material was loaded into the storage bin. The air pressure was adjusted to its maximum flow rate. The maximum flow rate was the rate that maximized DCDPS flow rate into the polymerization reactor, prior to the onset of clogging issues. After clogging onset, the DCDPS flow rate is decreased (or has a negligible increase) with an increase in air pressure.

For the batches tested, the batch consisting of DCDPS pastilles alone had significantly increased charge rates, relative to the batches consisting of a blend of DCDPS granules and pastilles. In particular, the charge rate of batches 5 and 6 were measured to be 3,750 and 3,650 pounds per hour ("lbs/hr"), while batch 7 had a charge rate of 5,800 lbs/hr. In other words, the batch having DCDPS pastilles (batch 7) had an increase charge rate of about 55% to about 59%, relative to the batches having a 1:1 weight ratio of DCDPS pastilles to DCDPS granules.

## Claims

1. A collection of pastilles comprising dichlorodiphenylsulfone ("DCDPS").

2. The collection of pastilles of claim 1 comprising an angle of repose from about 10° to about 80°, preferable 15° to about 45°.

3. The collection of pastilles of either one of claim 1 and 2, wherein the pastilles have an average height from about 1 mm to about 4 mm, preferably to about 3 mm , more preferably to about 2 mm.

4. The collection of pastilles of any one of claims 1 to 3, wherein the pastilles have an average width from about 1 mm to about 5 mm, preferably form about 2 mm to about 4mm.

5. The collection of pastilles of any one of claims 1 to 4, wherein at least about 90 percent, preferably at least about 95 percent, of the pastilles have a width greater than about 80 percent of the average width and less than about 120 percent of the average width.

6. The collection of pastilles according to any one of claims 1 to 5, wherein at least 90 percent, preferably at least about 95 percent, of the pastilles have a height that is greater than about 80 percent of the average height and less than about 120 percent of the average height.

7. The collection of pastilles according to any one of claims 1 to 6 having an average aspect ratio of from about 1.8 mm to about 2.2 mm.

8. The collection of pastilles according to any one of claims 1 to 7, comprising no more than about 5 wt.%, preferably no more than about 4 wt.%, more preferably no more than about 3 wt.%, still more preferably no more than about 2 wt.%, most preferably no more than about 1 wt.% of DCDPS powder, relative to the total weight of the DCDPS pastilles and DCDPS powder.

9. The collection of pastilles according to any one of claims 1 to 8, wherein the DCDPS is represented by the following formula: 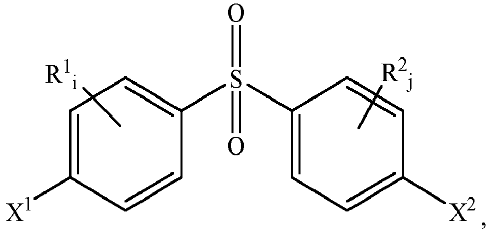 wherein R¹ and R², at each instance, are independently selected from the group consisting of a halogen, an alkyl, an alkenyl, an alkynyl, an aryl, an ether, a thioether, a carboxylic acid, an ester, an amide, an imide, an alkali or an alkaline earth metal sulfonate, an alkyl sulfonate, an alkali or alkaline earth metal phosphonate, an alkyl phosphonate, an amine and a quaternary ammonium; i and j are independently selected integers from 0 to 4, and X¹ and X² are independently selected halogens.

10. The collection of pastilles according to claim 9, wherein X¹ and X² are independently selected from Cl and F, preferably X¹ and X² are Cl.

11. The collection of pastilles according to any one of claims 1 to 10, wherein the collection of pastilles has a weight of at least about 1 kg, preferably at least about 10 kg, more preferably at least about 20 kg, still more preferably at least about 40 kg, most preferably at least about 100 kg.

12. A method of making the collection of pastilles of any one of claims 1 to 11, the method comprising depositing liquid DCDPS onto a surface, wherein the liquid DCDPS is at a temperature of from about Tm + 5° C to about Tm + 35° C; wherein the surface is at a temperature from about Tm - 80 ° C to Tm - 110 ° C, preferably to Tm - 100 ° C; and wherein Tm is the melting point of DCDPS.

13. A method of synthesizing a PAES polymer, the method comprising:
forming a reaction mixture comprising:
- the collection of DCDPS pastilles of anyone of claims 1 to 11 and
- a diol monomer represented by the following formula: 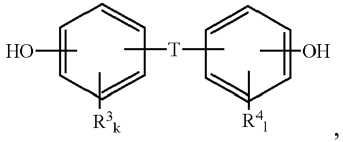
wherein R³ and R⁴, at each instance, are independently selected from a halogen, an alkyl, an alkenyl, an alkynyl, an aryl, an ether, a thioether, a carboxylic acid, an ester, an amide, an imide, an alkali or alkaline earth metal sulfonate, an alkyl sulfonate, an alkali or alkaline earth metal phosphonate, an alkyl phosphonate, an amine, and a quaternary ammonium; k and l are integers ranging from 0 to 4; and T is selected from the group consisting of a bond, a sulfone group [S(=O)2], and a group -C(R⁵)(R⁶)-, where R⁵ and R⁶ are independently selected from a hydrogen, a halogen, an alkyl, an alkenyl, an alkynyl, an ether, a thioether, a carboxylic acid, an ester, an amide, an imide, an alkali or alkaline earth metal sulfonate, an alkyl sulfonate, an alkali or alkaline earth metal phosphonate, an alkyl phosphonate, an amine, and a quaternary ammonium
heating the reaction mixture to a temperature between about 180° C to about 240 ° C.

14. The method of claim 13, wherein the PAES polymer is represented by the following formula, 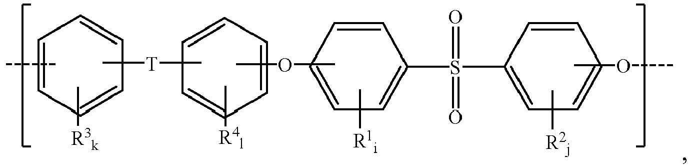 preferably the following formula
